# EUROPÄISCHE PATENTSCHRIFT

(11) **EP 2 922 496 B1**
(45) Veröffentlichungstag und Bekanntmachung des Hinweises auf die Patenterteilung: **05.08.2020**
(21) Anmeldenummer: 13798614.7
(22) Anmeldetag: 21.11.2013
(51) Int. Cl.: A61F 2/07

(54) **GEFÄSSIMPLANTAT MIT ASYMMETRISCHEN STENTFEDERN**
VASCULAR IMPLANT WITH ASYMMETRICAL STENT SPRINGS
IMPLANT VASCULAIRE À RESSORTS DE STENT ASYMÉTRIQUES

(30) Priorität: 21.11.2012 DE 102012111223
(43) Veröffentlichungstag der Anmeldung: 30.09.2015
(73) Patentinhaber: JOTEC GmbH, 72379 Hechingen (DE)
(72) Erfinder: FISCHER, Heike, 40670 Meerbusch (DE); MERZ, Juergen, 72336 Balingen (DE)
(74) Vertreter: Witte, Weller & Partner Patentanwälte mbB
(86) Internationale Anmeldenummer: PCT/EP2013/074366
(87) Internationale Veröffentlichungsnummer: WO 2014/079918

(56) Entgegenhaltungen:
- WO-A1-2006/125382
- WO-A2-2008/051543
- WO-A2-2012/061526
- US-A1- 2007 055 347
- US-A1- 2010 011 976
- US-A1- 2011 218 617

## Beschreibung

Die vorliegende Erfindung, die durch Anspruch 1 definiert wird, betrifft ein selbstexpandierendes Gefäßimplantat zur Implantation in ein Blutgefäß mit einem hohlzylindrischen Körper mit einem ersten und einem zweiten Ende und einer Längsrichtung, sowie mit in seiner Längsrichtung in einem Abstand hintereinander angeordneten, jeweils mäanderförmig umlaufenden Stentfedern und einem an den Stentfedern befestigten und diese verbindenden Implantatmaterial, wobei die Stentfedern lediglich über das Implantatmaterial und nicht untereinander verbunden sind, und wobei die umlaufenden Stentfedern abwechselnd in proximale und distale Richtung weisende Spitzbögen mit abwechselnden Scheitelpunkten und Tiefstpunkten aufweisen, welche über Schenkel miteinander verbunden sind, wobei ein in proximale Richtung x weisender höherer Spitzbogen einer ersten Stentfeder einem in distale Richtung y weisenden kürzeren Spitzbogen einer proximal nachgeordneten zweiten Stentfeder in einem Abstand und in einer gedachten Linie gegenüber liegt, die parallel zur Längsrichtung des Gefäßimplantats ist, und/oder wobei ein in proximale Richtung x weisender kürzerer Spitzbogen einer ersten Stentfeder einem in distale Richtung y weisenden längeren Spitzbogen einer in proximale Richtung x nachgeordneten zweiten Stentfeder in einem Abstand und in einer gedachten Linie gegenüber liegt, die parallel zur Längsrichtung des Gefäßimplantats ist.

Derartige Gefäßimplantate sind im Stand der Technik bspw. aus der DE 103 37 739.5 bekannt.

Allgemein ist es bekannt, intraluminale Gefäßimplantate, die auch als endovaskuläre Gefäßstents/-stentgrafts bezeichnet werden, zur Behandlung von Aneurysmen in Arterien zu implantieren. Unter einem Aneurysma versteht man dabei eine Ausweitung oder Aussackung eines arteriellen Blutgefäßes infolge angeborener oder erworbener Wandveränderungen. Die Aussackung kann dabei die Gefäßwand als Ganzes erfassen, oder, wie bei dem sogenannten falschen Aneurysma bzw. der sogenannten Dissektion, es tritt Blut aus dem Lumen des Gefäßes zwischen die Gefäßwandschichten und schert diese auseinander. Die Nichtbehandlung eines Aneurysma kann im fortgeschrittenen Stadium zu einer Ruptur der Arterie führen, mit der Folge, dass der Patient innerlich verblutet.

Die zur Behandlung derartiger Aneurysmen verwendeten Selbstexpandierenden Gefäßimplantate bestehen im Allgemeinen aus einem hohlzylindrischen Metallrahmen, dessen Mantelfläche mit einer Textil- oder Polymerfolie abgedeckt ist, so dass sich ein hohlzylindrischer Körper ergibt. Zur Implantation wird das Gefäßimplantat radial zusammengedrückt, so dass sich seine Querschnittsfläche deutlich verringert. Das Gefäßimplantat wird dann mit Hilfe eines Einführsystems in den Bereich des Aneurysma gebracht, wo sie freigesetzt wird. Auf Grund der Federwirkung des Metallrahmens expandiert das Gefäßimplantat wieder in seine ursprüngliche Form und spannt dabei seine Mantelfläche auf, die sich proximal und distal von dem Aneurysma innen in dem Blutgefäß verklemmt. Auf diese Weise fließt das Blut jetzt durch das Gefäßimplantat und eine weitere Belastung der Aussackung wird verhindert.

Der Metallrahmen solcher Gefäßimplantate besteht in der Regel bspw. aus einem Drahtgeflecht oder aus hintereinander angeordneten, mäanderförmig umlaufenden, sogenannten Stentfedern, die ggf. über Verbindungsstützen aus Draht miteinan-der verbunden sind, oder die lediglich über das Implantatmaterial miteinander verbunden sind. Das Drahtgeflecht bzw. die Stentfedern sind üblicherweise aus einem Shape-Memory-Material, in der Regel aus Nitinol, wodurch die Stentfedern nach Einbringung in ein Gefäß zur Freisetzung wieder in den expandierten Zustand übergehen und das Gefäßimplantat dadurch "aufspannen".

Die WO 2008/051543 A2 offenbart einen Stentgraft mit über ein Graftmaterial verbundenen und hintereinander angeordneten Stentfedern. Die Stentfedern der hierin offenbarten Stentgrafts weisen dabei entweder gleichmäßige Amplituden der mäanderförmig umlaufenden Spitzbögen auf, oder aber eine in umlaufender Richtung regelmäßig verkleinernde Amplitude. Dieses Dokument offenbart die Merkmale des einleitenden Teils des Anspruchs 1.

Die WO 2008/051543 A2 offenbart einen Stentgraft mit über ein Graftmaterial verbundenen und hintereinander angeordneten Stentfedern. Die Stentfedern der hierin offenbarten Stentgrafts weisen dabei entweder gleichmäßige Amplituden der mäanderförmig umlaufenden Spitzbögen auf, oder aber eine in umlaufender Richtung regelmäßig verkleinernde Amplitude.

Die US 2007/0055347 offenbart ein selbstexpandierendes Gefäßimplantat mit über ein Graftmaterial verbundenen und hintereinander angeordneten Stentfedern. Die Spitzbögen der Stentfedern weisen hierbei abwechselnd in proximale und distale Richtung, wobei die Scheitelpunkte der Spitzbögen die gleiche Höhe aufweisen, also auf einer Ebene liegen, die senkrecht zur Stentachse steht.

Die im Stand der Technik bekannten Gefäßimplantate, insbesondere diejenigen, die mäanderförmig umlaufende Stentfedern aufweisen, die entweder lediglich über Implantatmaterial und damit indirekt miteinander verbunden sind, oder aber die zusätzlich über Verbindungsschenkel oder -stützen untereinander verbunden sind, besteht bei der Einbringung in ein Gefäß, und insbesondere dann wenn das Gefäßimplantat in oder über einen gebogenen Abschnitt eines Gefäßes eingebracht wird, die Gefahr einer Knickbildung. Diese Knickbildung tritt insbesondere dann auf, wenn sich das Gefäßimplantat dem natürlichen Verlauf des gebogenen Gefäßes anpassen soll und der Bogen im Bereich von zwei oder mehreren hintereinander angeordneten Stentfedern verlaufen werden soll: In diesem Bereich liegen sich üblicherweise ein Tiefstpunkt der einen Stentfeder und ein Scheitelpunkt der distal nachgeschalteten Stentfeder gegenüber, so dass aufgrund des kleinen Zwischenraums, der zwischen diesen beiden Spitzen liegt und nur durch das Implantatmaterial gebildet wird, das Gefäßimplantat hier leicht einknickt.

Eine solche Knickbildung ist äußerst nachteilig, da bei einer Knickbildung ein regelmäßiger Blutfluss durch das Gefäß nicht mehr gewährleistet ist, und es bspw. zu Stauungen und Verwirbelungen des Blutes innerhalb des Gefäßimplantats oder einem Gefäßverschluss kommen kann.

Aufgabe der vorliegenden Erfindung ist es daher, ein Gefäßimplantat bereitzustellen, mit dem die Gefahr einer Knickbildung des Gefäßimplantats vermieden oder zumindest weitestgehend verringert werden kann, wobei die Längsstabilität des Gefäßimplantats erhalten bleibt.

Erfindungsgemäß wird diese Aufgabe durch eine Weiterbildung des eingangs genannten Gefäßimplantats gelöst, bei welchem zumindest zwei in Längsrichtung hintereinander angeordnete Stentfedern jeweils Schenkel mit unterschiedlichen Längen aufweisen zur Ausbildung von umlaufend aufeinanderfolgenden unterschiedlich hohen Spitzbögen, mit höheren und kürzeren Spitzbögen, wobei zumindest drei in umlaufender Richtung unmittelbar aufeinanderfolgende Schenkel einer Stentfeder jeweils unterschiedliche Längen aufweisen, wie in den beiliegenden Ansprüchen definiert..

Durch die Ausbildung unterschiedlicher Schenkellängen und die dadurch gebildeten unterschiedlich hohen, abwechselnd in proximale und distale Richtung weisenden Spitzbögen ist der Verlauf einer Stentfeder insgesamt asymmetrisch. Die Stentfedern, zumindest zwei, vorzugswiese alle Stentfedern eines Gefäßimplantats, weisen somit unregelmäßige "Amplituden", d.h. Spitzbögen auf, die aufgrund der unterschiedlichen langen Schenkel unterschiedlich hoch sind. Es werden also erfindungsgemäß in umlaufender Richtung der Stentfeder abwechselnd höhere und kürzere Spitzbögen gebildet. Dabei sollen die Begriffe "unterschiedlich hoch" und "höhere" und "kürzere" sich auf das Verhältnis der Spitzbögen untereinander beziehen. Der höchste Spitzbogen einer Stentfeder besitzt daher einen Schenkel, der bezüglich anderer Schenkel der Stentfeder derselben Stentfeder am Längsten ist. Die Länge des längsten Schenkels einer Stentfeder gibt damit auch die Höhe der Stentfeder, also die Stentfederhöhe, vor. Es versteht sich dabei, dass - obgleich sich die Längen unmittelbar aufeinanderfolgender Schenkel unterscheiden - eine Stentfeder insgesamt mehrere Schenkel mit gleicher Länge aufweisen kann, deren Folge aber definitionsgemäß durch Schenkel anderer Längen unterbrochen wird.

Durch die Kombination dieser asymmetrischen Stentfedern mit der Anordnung zumindest zweier hintereinander angeordneter Stentfedern derart, dass ein höherer, in proximale Richtung weisender Spitzbogen einer ersten Stentfeder einem kürzeren in distale Richtung weisenden Spitzbogen einer in distaler Richtung nachgeordneten zweiten Stentfeder gegenüber liegt, wird vorteilhafterweise erreicht, dass ein Biegen des erfindungsgemäßen Gefäßimplantats in einem Gefäß nicht zu einer Knickbildung führt bzw. diese weitestgehend vermieden oder erst bei deutlich kleineren Radien auftreten kann und das Risiko stark abgeschwächt wird. Dadurch, dass ein in proximale Richtung weisender höherer Spitzbogen einer ersten Stentfeder einem in distale Richtung weisenden kürzeren Spitzbogen einer proximal nachgeordneten zweiten Stentfeder in einem Abstand und in einer gedachten Linie, die parallel zur Längsrichtung des Gefäßimplantats ist, gegenüber liegt, ist die Längssteifigkeit bei hoher Flexibilität gut. Lange und kurze Scheitelpunkte greifen quasi bei Biegung ineinander. Dadurch ist der Stentgraft auch in drei Dimensionen sehr flexibel.

Die abwechselnd in proximale und distale Richtung weisenden Spitzbögen können, anders gesprochen, auch als "Wellenberge" und "Wellentäler" bezeichnet werden, wobei ein "Wellenberg" einen in proximale Richtung weisenden Spitzbogen und ein "Wellental" einen in distale Richtung weisenden Spitzbogen bezeichnet. Bei der genannten Ausführungsform wird also ein asymmetrischer mäanderförmiger Verlauf der umlaufenden Stentfedern erreicht, bei welchem jeweils zwei aufeinanderfolgende Wellenberge - bzw. in proximale Richtung weisende Spitzbögen - unterschiedliche Höhen besitzen, und damit auch die jeweils dazwischen liegenden Wellentäler - bzw. in distale Richtung weisenden Spitzbögen - unterschiedliche Tiefen. Insgesamt folgt also in umlaufender Richtung abwechselnd auf einen höheren Wellenberg/Scheitelpunkt ein Wellenberg/Scheitelpunkt, der niedriger bzw. weniger hoch ist als der vorherige Wellenberg/Scheitelpunkt, und auf diesen Wellenberg/Scheitelpunkt wieder ein Wellenberg/Scheitelpunkt, der höher ist als der Wellenberg/Scheitelpunkt unmittelbar davor, usw., wobei zwischen den unterschiedlich hohen Wellenbergen/Scheitelpunkten jeweils unterschiedlich tiefe Wellentäler/Tiefstpunkte liegen: Auch bei den Wellentälern/Tiefstpunkten folgt in umlaufender Richtung abwechselnd auf ein tieferes Wellental/Tiefstpunkt wieder ein Wellental/Tiefstpunkt, das weniger tief ist als das in umlaufender Richtung unmittelbar vorher angeordnete Wellental/Tiefstpunkt, und auf dieses Wellental/Tiefstpunkt wieder ein Wellental/Tiefstpunkt, das tiefer ist als das davor. Anders gesprochen besitzt eine Stentfeder also Spitzbögen mit mindestens zwei oder drei unterschiedlich tiefen Tiefstpunkten, wobei sich die unterschiedliche Tiefen auf eine senkrecht zur Längsrichtung des hohlzylindrischen Grundkörpers umlaufende, gedachte Linie beziehen, welche die tiefsten Tiefstpunkte miteinander verbindet. Definitionsgemäß liegen damit weniger tiefe Tiefstpunkte nicht auf dieser gedachten Linie, und gleiches gilt wiederum für die Scheitelpunkte.

Ebenfalls anders gesprochen, sind gemäß des erfindungsgemäßen Gefäßimplantats zumindest zwei in Längsrichtung hintereinander angeordnete Stentfedern in Bezug auf die Längsrichtung des hohlzylindrischen Grundkörpers und in Bezug aufeinander derart angeordnet sind, dass die Tiefstpunkte der Spitzbögen einer ersten Stentfeder jeweils den Scheitelpunkten von Spitzbögen einer in Längsrichtung des Gefäßimplantats der ersten Stentfeder nachgeordneten zweiten Stentfeder in einem Abstand und in einer gedachten Linie, die parallel zur Längsrichtung des Gefäßimplantats ist, gegenüber liegen, und zwar derart, dass ein tiefster Tiefstpunkt eines Spitzbogens der ersten Stentfeder einem Scheitelpunkt der distal nachgeordneten zweiten Stentfeder in einem Abstand gegenüber liegt, wobei dieser Scheitelpunkt weniger hoch ist als ein höchster Scheitelpunkt der zweiten Stentfeder; entsprechend kommt damit bei dieser Ausführungsform immer auch ein weniger tiefer Tiefstpunkt - also ein Tiefstpunkt, der weniger tief ist als ein tiefster Tiefpunkt der ersten Stentfeder - in einem Abstand zu und gegenüber einem höchsten Scheitelpunkt der distal nachgeordneten zweiten Stentfeder zu liegen, welcher höchste Scheitelpunkt höher ist als ein weniger hoher Scheitelpunkt der zweiten Stentfeder.

Es versteht sich, dass eine Stentfeder insgesamt unterschiedliche Schenkel-Längen ihrer Spitzbögen aufweisen kann, oder aber eben lediglich drei oder mehr als drei unterschiedlich lange. Auch können die relativen Abstände zwischen einem Scheitelpunkt und einem Tieftspunkt oder zwischen allen Scheitelpunkten und Tiefstpunkten einer Stentfeder unterschiedlich sein.

Mit dem erfindungsgemäßen Gefäßimplantat wird erreicht, dass zwischen den benachbart gegenüber liegenden Spitzbögen zwei verschiedene Biegeebenen vorliegen, wodurch das Gefäßimplantat deutlich flexibler wird, seine Längsstabilität aber beibehält. Gleichzeitig kann es ohne abzuknicken in mehrere Ebenen gebogen werden.

In Zusammenwirkung mit den unterschiedlichen Schenkellängen verleiht diese Phasenanordnung mindestens zweier, vorzugsweise aller Stentfedern des Gefäßimplantats diesem ein asymmetrisches Muster, wodurch ein Biegen des erfindungsgemäßen Gefäßimplantats in einem Gefäß nicht zu einer Knickbildung führt bzw. diese weitestgehend vermieden oder erst bei deutlich kleineren Radien auftreten kann und das Risiko stark abgeschwächt wird.

Die der Erfindung zugrunde liegende Aufgabe wird auf diese Weise vollkommen gelöst.

Vorliegend wird dabei unter einer "Stentfeder" wie eingangs diskutiert, jedes ringförmige Element verstanden, das sich aufgrund dessen Materials komprimieren lässt, und sich nach Entfernung des Kompressionsdrucks wieder federartig expandieren kann. Unter "mäanderförmig" wird vorliegen jeder wellen-, schlingen- bzw. schleifenförmige Verlauf" der Stentfeder bzw. des Stentdrahtes verstanden, wobei jede Stentfeder einstückig, d.h. aus einem mäanderförmig umlaufenden Stentfederring gebildet ist. Entsprechend ist eine "mäanderförmig umlaufende Stentfeder" ist in diesem Zusammenhang ein sich federartig expandierendes und komprimierbares ringförmiges Stentelement, das einen wellen-, schleifen- oder schlingenartigen Verlauf besitzt, wobei sich Wellenberg und Wellental, abwechseln.

Bei dem erfindungsgemäßen Gefäßimplantat ist dabei ein Spitzbogen jeweils aus zwei Schenkeln und einem zwischen den Schenkeln liegenden Scheitelpunkt (bei einem in proximale Richtung weisenden Spitzbogen) bzw. Tiefstpunkt (bei einem in distale Richtung weisenden Spitzbogen) gebildet.

Aufgrund der unterschiedlich langen Schenkel ergeben sich unterschiedliche hohe Spitzbögen, wobei deren Höhe bezüglich einer gedachten Linie bestimmt ist, die in umlaufender Richtung der Stentfeder und senkrecht zur Längsrichtung des Gefäßimplantats durch den/die höchsten Scheitelpunkte der in proximale Richtung weisenden Spitzbögen verläuft. Aufgrund dieser Ausgestaltung und Definition liegen stets Scheitelpunkte vor, die unterhalb dieser gedachten Linie durch die höchsten Scheitelpunkte liegen, und damit Scheitelpunkte darstellen, die kürzer als die höchsten Scheitelpunkte sind. Analog gilt dies auch bezüglich der in distale Richtung weisenden Spitzbögen bzw. der Tiefstpunkte: auch hier ist eine gedachte Linie in umlaufender Richtung einer Stentfeder durch den/die höchsten Scheitelpunkt(e) der in distale Richtung weisenden Spitzbögen gezogen, so dass höhere und kürzere Scheitelpunkte der in distale Richtung weisenden Spitzbögen vorliegen.

Beispielhafte Höhen der unterschiedlichen Spitzbögen liegen bspw. im Bereich von 4 bis 18 mm, vorzugsweise ca. 8 mm bis 14 mm für die höchsten Spitzbögen, also für die Spitzbögen, die höher sind als kürzere Spitzbögen, und von 4 bis 10 mm vorzugsweise 6 mm bis 8 mm, für die kürzeren Spitzbögen. Dabei wird dem Fachmann klar sein, dass bei einer Stentfeder einerseits Spitzbögen mit zumindest zwei oder drei oder vier oder mehr unterschiedlich hohen Spitzbögen vorliegen können. Bei Vorliegen dreier unterschiedlich hoher Spitzbögen weist eine Stentfeder also drei unterschiedliche Höhen für die Spitzbögen auf, also zumindest einen ersten höheren Spitzbogen, dessen Höhe am höchsten ist, zumindest einen zweiten Spitzbogen, dessen Höhe kürzer ist als die des ersten Spitzbogens, und einen dritten Spitzbogen, dessen Höhe wiederum kürzer als die des zweiten Spitzbogens ist, etc. Beispielhafte Höhen, die vorliegend lediglich exemplarischen Zwecken dienen und nicht limitierend sein sollen, sind bspw. 10 mm (höhere Spitzbögen) und 8 mm (kürzere Spitzbögen); 12 mm (höhere Spitzbögen) und 8 mm (kürzere Spitzbögen); 12 mm (höhere Spitzbögen) und 9 mm (kürzere Spitzbögen); 12 mm (höhere Spitzbögen) und 10 mm (kürzere Spitzbögen); 16 mm (höhere Spitzbögen) und 14 mm (kürzere Spitzbögen), 16 mm (höhere Spitzbögen) und 13 mm (kürzere Spitzbögen); 16 mm (höhere Spitzbögen), 12 mm (kürzere Spitzbögen) und 10 mm (noch kürzere Spitzbögen); 10 mm (höhere Spitzbögen) und 8 mm (kürzere Spitzbögen); 12 mm (höhere Spitzbögen), 10 mm (kürzere Spitzbögen) und 8 mm (noch kürzere Spitzbögen).

Vorliegend und durchgehend durch die Beschreibung soll dabei der Begriff "ca." bedeuten, dass bei den genannten Bereichs- und Zahlenangaben auch solche noch mit umfasst sein sollen, die aufgrund von Messunterschieden oder Toleranzen für den Fachmann noch mit umfasst sind, und die geeignet sind, die der Erfindung zu Grunde liegende Aufgabe zu lösen bzw. hierzu beizutragen.

Vorliegend wird dabei mit Bezug auf das Gefäßimplantat allgemein mit dem Ausdruck "proximal" diejenige Position, Richtung oder ein Abschnitt oder Ende einer Komponente des Gefäßimplantats bezeichnet, die/der am nächsten zum Herzen des zu behandelnden Patienten liegt.

Entsprechend wird vorliegend mit "distal" diejenige Position, Richtung oder ein Abschnitt oder Ende einer Komponente des erfindungsgemäßen Gefäßimplantats bezeichnet, die vom Herzen eines Patienten weiter/am weitesten entfernt ist/führt.

Entsprechend sind vorliegend das/die "proximale" und die "distale" Ende/ Öffnung des Gefäßimplantats die Enden/Öffnungen, durch die hindurch der Blutfluss durch den hohlzylindrischen Körper des Gefäßimplantats gewährleistet wird: Wenn das erfindungsgemäße Gefäßimplantat in einem Blutgefäß wie beispielsweise der Aorta implantiert ist, fließt also das vom Herzen kommende Blut durch das/die proximale Ende/Öffnung des Gefäßimplantats, und verlässt das Gefäßimplantat durch deren distale Öffnung.

Definitionsgemäß sind die Stentfedern nicht direkt miteinander verbunden, und weisen untereinander keine verbindenden Schenkel oder Streben oder ähnliche Verbindungselemente auf. Die Stentfedern sind lediglich über das Implantatmaterial, an welches die Stentfedern angebracht sind, miteinander verbunden, wodurch eine "indirekte Verbindung" zwischen den Stentfedern geschaffen wird. Hintereinander in Längsrichtung des Gefäßimplantats angeordnete Stentfedern liegen also in einem Abstand zu einander und berühren sich im ungebogenen Zustand des Gefäßimplantats nicht. Der Abstand zwischen den Stentfedern kann variieren und hängt insbesondere vom Einsatzzweck und der Beschaffenheit des Gefäßimplantats ab; beispielhafte Abstände liegen zwischen 1 mm (bei zueinander verdrehten langen Spitzen auch 0 mm) und 20 mm, vorteilhafterweise bei 1 bis 4 mm.

Ferner wird vorliegend unter "Stent" jede Vorrichtung oder eine Struktur bezeichnet, die aufgrund eines elastischen Metallrahmens eines Implantats eine Expansionskraft und/oder eine stützende Funktion verleiht.

Der Ausdruck "Stentgraft" soll vorliegend - wie auch im Stand der Technik - ein Implantat bedeuten, die einen Stent sowie ein damit verbundenes Implantat("graft")-Material aufweist, das ein Lumen durch zumindest einen Abschnitt des Implantats bildet.

Mit dem erfindungsgemäßen Gefäßimplantat wird darüber hinaus auch bewirkt, dass das Implantat auch im gebogenen Zustand rotiert werden kann, so dass auch dreidimensionale Biegungen hier realisiert werden können, ohne dass das Produkt bzw. Implantat im Gefäß knickt.

Bei den im Stand der Technik bekannten Gefäßimplantaten mit hintereinander angeordneten Stentfedern ist eine Rotation in gebogenem Zustand nicht möglich, da hier aufgrund der symmetrischen Stentfedern Knicke auftreten. Die Knickbildung bei den im Stand der Technik bekannten Stentfedern liegt damit an deren symmetrischen mäandrischen Ausbildung und an der symmetrischen Anordnung der Stentfedern über die Längsachse hintereinander. Durch diese symmetrische Anordnung der Stentfedern in Längsrichtung kommt es bei einer Biegung des Gefäßimplantats aus dem Stand der Technik zur oben geschilderten Knickbildung.

Ferner wird mit dem erfindungsgemäßen Gefäßimplantat bzw. der alternierende Lage der Spitzbögen der Stentfedern beim Laden des Gefäßimplantats in ein Einführsystem, bei welchem Vorgang insbesondere die Bögen der Stentfedern platzraubend sind, vorteilhafterweise erreicht, dass das Gefäßimplantat deutlich enger komprimiert und dadurch in kleine Einführkatheter geladen werden kann.

Gemäß des erfindungsgemäßen Gefäßimplantats besitzen zumindest drei in umlaufender Richtung einer Stentfeder unmittelbar aufeinanderfolgende Schenkel unterschiedliche Längen, wobei der erste Schenkel einen ersten Tiefstpunkt mit einem ersten Scheitelpunkt verbindet, der zweite Schenkel den ersten Scheitelpunkt mit einem in umlaufender Richtung folgenden zweiten Tiefstpunkt, und der dritte Schenkel den zweiten Tiefstpunkt mit einem in umlaufender Richtung folgenden zweiten Scheitelpunkt.

Bei einer weiteren Ausführungsform ist bevorzugt, wenn in umlaufender Richtung der Stentfeder jeweils ein höherer mit einem kürzeren in proximale Richtung x weisenden Spitzbogen alterniert, und jeweils ein höherer mit einem kürzeren in distale Richtung y weisender Spitzbogen.

Bei einer noch weiteren Ausführungsform ist bevorzugt, wenn bei zumindest einer Stentfeder in umlaufender Richtung die in proximale und/oder distale Richtung x oder y weisenden Spitzbögen derart ausgebildet sind, dass auf einen hohen Spitzbogen ein kürzerer Spitzbogen folgt, und auf den kürzeren Spitzbogen ein Spitzbogen, der kürzer ist als der vorher angeordnete kürzere Spitzbogen, und auf diesen wieder ein hoher Spitzbogen, usw. Bei dieser Ausführungsform folgt damit praktisch ein "Dreiermuster" aus einem hohen, kürzeren und noch kürzerem Spitzbogen, einem eben solchen Dreiermuster, wodurch definitionsgemäß ebenfalls eine asymmetrische Stentfeder bereitgestellt wird.

Gemäß einer noch weiteren Ausführungsform kann vorgesehen sein, wenn bei zumindest einer Stentfeder in umlaufender Richtung die in proximale Richtung weisenden Spitzbögen alle die gleiche Höhe aufweisen, welche sich mit in distale Richtung weisenden Spitzbögen unterschiedlicher Höhe abwechseln.

Mit den oben ausgeführten verschiedenen Ausführungsformen können die erfindungsgemäßen asymmetrische Stentfedern und asymmetrische Stentfeder-Anordnung erreicht werden.

Gemäß einer weiteren Ausführungsform ist bevorzugt, wenn sich der Abstand z1 eines ersten Scheitelpunkts zu einem auf den ersten Scheitelpunkt in umlaufenden Richtung folgender ersten Tiefstpunkt zumindest einer Stentfeder unterscheidet von dem Abstand z2 des ersten Tiefstpunkts zu einem auf den ersten Tiefstpunkt in umlaufender Richtung folgenden zweiten Scheitelpunkt derselben Stentfeder.

Diese Ausführungsform hat den Vorteil, dass eine zusätzliche Asymmetrie geschaffen werden kann, mit Hilfe derer eine Knickbildung des Gefäßimplantats weiter vermindert werden kann.

Gemäß einer Ausführungsform des erfindungsgemäßen Gefäßimplantats ist bevorzugt, wenn das erfindungsgemäß selbstexpandierende Gefäßimplantat mindestens drei, in Längsrichtung hintereinander angeordnete Stentfedern aufweist, die nicht unmittelbar untereinander, sondern lediglich über das Implantatmaterial miteinander verbunden sind.

Gemäß einer weiteren Ausführungsform ist bevorzugt, wenn das erfindungsgemäße Gefäßimplantat zwischen drei und zehn, vorzugsweise, drei, vier, fünf, sechs, sieben, acht, neun oder zehn hintereinander angeordnete Stentfedern aufweist.

Die Anzahl der Stentfedern hängt dabei von der notwendigen Länge des einzusetzenden Gefäßimplantats ab, bzw. von den zu überbrückenden Gefäßdefekten eines Patienten.

Gleichermaßen hängt die Anzahl der Scheitelpunkte und Tiefstpunkte eines erfindungsgemäßen Gefäßimplantats, bzw. die alternierend in proximale und distale Richtung weisenden Spitzbögen, von dem Durchmesser des zu behandelnden Gefäßes bzw. Gefäßabschnitts ab. So ist bspw. bei einem Gefäßimplantat, das bei einem erwachsenen Patienten im arteriellen Gefäßsystem eingesetzt wird, bevorzugt, wenn dieses zwischen sechs und zwölf in proximale und zwischen sechs und zwölf in distale Richtung weisende Spitzbögen aufweist.

Es versteht sich, dass der behandelnde Arzt anhand des Durchmessers des Gefäßes, sowie des Umfanges der zu behandelnden Gefäßregion Maße vorgeben kann, anhand derer die optimale Stentfederanzahl, sowie Spitzbogen- bzw. Scheitel- und Tiefstpunktanzahl bestimmt werden kann.

Bei dem erfindungsgemäßen Gefäßimplantat ist insgesamt vorgesehen, dass die Stentfedern in einem bestimmten Abstand zueinander an dem Implantatmaterial angebracht sind. Die Stentfedern werden dabei vorzugsweise angenäht bzw. angeklebt. Der Abstand der hintereinander in Längsrichtung angeordneten Stentfedern ist dabei derart, dass das Gefäßimplantat insgesamt eine hinreichende Stabilität bei gleichzeitiger guter Biegsamkeit besitzt. So beträgt der Abstand zwischen zwei in Längsrichtung hintereinander angeordneten Stentfedern, gemessen an dem tiefsten Tiefstpunkt der ersten Stentfeder zu dem höchsten Scheitelpunkt der distal nachgeordneten zweiten Stentfeder - wobei der tiefste Tiefstpunkt und der höchste Scheitelpunkt nicht zwingendermaßen direkt gegenüberliegen - von zwischen ca. 0 mm bis ca. 5 mm.

Gemäß einer bevorzugten Ausführungsform des erfindungsgemäßen selbstexpandierenden Gefäßimplantats bestehen die Stentfedern aus einem selbstexpandierenden Material, vorzugsweise Nitinol.

Nitinol ist das gegenwärtig bei selbstexpandierenden Gefäßimplantaten, bei denen Stentfedern eingesetzt werden, das am häufigsten eingesetzte Material. Nitinol hat den Vorteil, dass es "Shape-Memory"-Eigenschaften aufweist, was bedeutet, dass Stentfedern aus Nitinol in expandiertem Zustand gefertigt werden, zum Einführen in das Gefäß komprimiert werden können, und nach Entfernen eines Kompressionsmittels, in der Regel einer Rückzugshülle, sich die Stentfedern aufgrund der "Shape-Memory"-Eigenschaften wieder in den expandierten Ausgangszustand selber zurück überführen. Dadurch schmiegt sich das Gefäßimplantat insgesamt an die Gefäßwand an und wird aufgrund der Federwirkung der Stentfedern an die Gefäßwand angedrückt, so dass es im Gefäß sicher verankert wird.

Wie bereits weiter oben erwähnt, ist dabei insgesamt bevorzugt, wenn die Stentfedern an das Implantatmaterial in einem Abstand voneinander und in Längsrichtung des Gefäßimplantats hintereinander angenäht sind.

Diese Maßnahme hat den Vorteil, dass die Stentfedern individualisiert und sogar in unterschiedlichen Abständen zueinander am Implantatmaterial befestigt werden können, so dass bspw. zwei hintereinander angeordnete Stentfedern einen größeren Abstand voneinander aufweisen als weiter distal oder weiter proximal gelegene weitere zwei Stentfedern. Auf diese Weise kann das Gefäßimplantat insgesamt dem zu behandelnden Gefäßabschnitt angepasst werden, wobei das Gefäßimplantat über seine Längsrichtung und/oder über seinen Umfang bspw. noch Fenestrierungen aufweisen kann, welche Öffnungen für abgehende Gefäße in dem zu behandelnden Bereich darstellen. Durch das Vorsehen von Fenestrierungen im Implantatmaterial - meist in Verbindung mit von dem Gefäßimplantat abgehenden Seitenästen - wird sichergestellt, dass auch nach Einbringung des Gefäßimplantats in ein Gefäß die Blutversorgung des in diesem Bereich abzweigenden Gefäßes sichergestellt ist.

Dabei versteht sich, dass die Fenestrierung im Implantatmaterial zwischen zwei hintereinander angeordneten Stentfedern vorgesehen ist. Dabei kann entweder die Fenestrierung dem Abstand der beiden Stentfedern angepasst sein, oder aber der Abstand der beiden Stentfedern voneinander der Größe der Fenestrierung. Es versteht sich auch, dass mehrere Fenestrierungen umlaufend auf dem Implantatmaterial zwischen zwei hintereinander angeordneten Stentfedern vorgesehen sein können, sowie mehrere Fenestrierungen verteilt über die Längsrichtung des Gefäßimplantats.

Dem Fachmann wird klar sein, an welchen Stellen des erfindungsgemäßen Gefäßimplantats die Fenestrierungen vorgesehen sein müssen, und wird dies in Abstimmung mit der jeweils spezifischen Gefäßbeschaffenheit vorsehen können.

Insgesamt ist bei dem erfindungsgemäßen Gefäßimplantat bevorzugt, wenn das Implantatmaterial ein Material aufweist, das ausgewählt ist aus Polyester, Polyurethan, Polystyrol, Polytetrafluorethylen oder ultrahochmolekulargewichtiges Polyethylen (UHMPE), oder Mischungen davon.

Der Einsatz dieser Materialien ist in der Medizin insbesondere auch für Gefäßimplantate bereits erprobt, so dass der Fachmann anhand seiner Kenntnisse geeignete Materialien für den spezifischen Einsatz des Gefäßimplantats auswählen kann.

Die vorliegende Erfindung betrifft daher auch eine weitere Ausführungsform des erfindungsgemäßen Gefäßimplantats, die derart ausgebildet ist, dass sie neben dem zumindest einen hohlzylindrischen Körper noch zumindest einen abzweigenden Seitenkörper aufweist, der von dem hohlzylindrischen Körper des Gefäßimplantats abzweigt. Der Seitenkörper ist dabei selber ein hohlzylindrischer Körper aus hintereinander angeordneten Stentfedern mit einem diese verbindenden Implantatmaterial, wobei die Seitenkörper für abgehende Gefäße vorgesehen sind. Die Seitenkörper weisen in der Regel einen kleineren Durchmesser auf als der "Grundkörper" des Gefäßimplantats.

Der zumindest eine Seitenkörper kann dabei selber aus einem gestenteten Abschnitt - d.h. einem Abschnitt, der Stentfedern oder ein Stent-Drahtgeflecht aufweist - bestehen oder einen solchen aufweisen, oder auch einen ungestenteten, lediglich aus Implantatmaterial bestehenden Abschnitt aufweisen oder aus einem solchen bestehen. Die Materialien, aus denen der Seitenkörper besteht, sind in der Regel auch diejenigen, aus denen der Grundkörper gefertigt ist.

Bei dem erfindungsgemäßen Gefäßimplantat kann ferner in einer besonderen Ausführungsform bevorzugt sein, wenn das Gefäßimplantat über seine Längsrichtung hinweg unterschiedliche Durchmesser aufweist.

Diese Ausführungsform hat den Vorteil, dass sie bspw. in sich verengenden bzw. verjüngenden Gefäßen eingesetzt werden kann oder aber in Gefäßen, die sich abzweigen, wie bspw. in der Bifurkation der Aorta.

Weitere Vorteile und Merkmale ergeben sich aus der nachfolgenden Beschreibung und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird in Bezug auf diese nachstehend näher beschrieben. Es zeigen:
- Fig. 1: eine beispielhafte Ausführungsform eines Gefäßimplantats des Standes der Technik in nicht gebogener Form;
- Fig. 2a: eine Ausführungsform des erfindungsgemäßen Gefäßimplantats in nicht gebogenem Zustand;
- Fig. 2b: einen vergrößerten Ausschnitt des erfindungsgemäßen Gefäßimplantats aus Fig. 2a;
- Fig. 3a: die Darstellung des beispielhaften Ausführungsbeispiels des Gefäßimplantats des Standes der Technik in gebogenem Zustand;
- Fig. 3b: das Ausführungsbeispiel des erfindungsgemäßen Gefäßimplantats aus Fig. 2a ebenfalls in gebogenem Zustand;
- Fig. 4: eine schematische Darstellung einer beispielhafter Ausbildung und Anordnung zweier hintereinander angeordneten Stentfedern eines erfindungsgemäßen Gefäßimplantats, mit A: zwei unterschiedlichen Höhen für die in die proximale oder distale Richtung weisenden Spitzbögen; und mit B: jeweils drei unterschiedlichen Höhen für die in die proximale oder distale Richtung weisenden Spitzbögen; und
- Fig. 5: eine schematische Darstellung beispielhafter Verläufe von Stentfedern, zur Verwendung in erfindungsgemäßen Gefäßimplantaten.

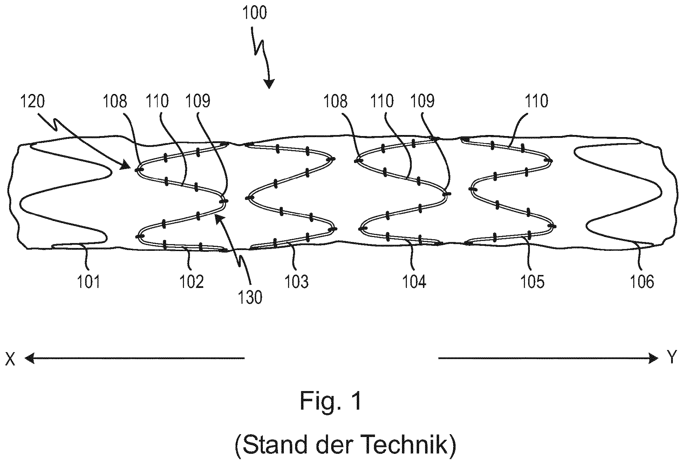

Fig. 1 zeigt ein selbstexpandierendes Gefäßimplantat, wie es im Stand der Technik bekannt ist. Dieses Gefäßimplantat 100 weist in Längsrichtung hintereinander angeordnete Stentfedern 101, 102, 103, 104, 105, 106 auf, die jeweils Scheitelpunkte 108 und Tiefstpunkte 109 aufweisen, bzw. in die proximale Richtung x weisende Spitzbögen120 und in die distale Richtung y weisende Spitzbogen 130.

Wie Fig. 1 zu entnehmen ist, sind die Scheitelpunkte 108 jeweils einer Stentfeder 101, 102, 103, 104, 105, 106 über Schenkel 110 mit Tiefstpunkten 109 derselben Stentfeder 101, 102, 103, 104, 105, 106 verbunden, und die Tiefstpunkte wiederum über Schenkeln 110 mit dem darauf folgenden Scheitelpunkt 108. In Fig. 1 ist ferner zu erkennen, dass der zwischen Scheitelpunkt und Tiefstpunkt abwechselnde Verlauf insgesamt zu einem symmetrischen mäanderförmigen Verlauf der Stentfeder 101, 102, 103, 104, 105, 106 führt. Die Stentfedern 101 bis 106 sind an ein Implantatmaterial 112 angebracht, und zwar derart, dass die Stentfedern 101 bis 106 symmetrisch angeordnet sind: zum einen weisen die Schenkel aller Stentfedern 101, 102, 103, 104, 105, 106, die jeweils Scheitelpunkt 108, Tiefstpunkt 109, Scheitelpunkt 108, Tiefstpunkt 109 etc. einer Stentfeder 101, 102, 103, 104, 105, 106 miteinander verbinden, die gleichen Längen auf, so dass sich die Scheitelpunkte 108 und Tiefstpunkte 109 umlaufend jeweils auf der gleichen Höhe - also sozusagen in der gleichen Amplitude - befinden. Zum anderen sind die hintereinander in Längsrichtung des Gefäßimplantat angeordneten Stentfedern bei dem Gefäßimplantat des Standes der Technik derart in einem Abstand zu einander angeordnet, dass ein Tiefstpunkt 109 der Stentfeder 101 dem Scheitelpunkt 108 der distal gelegenen Stentfeder 102 gegenüberliegt, ohne dass diese sich unmittelbar berühren oder direkt miteinander verbunden wären. Entsprechend ist auch wiederum ein Tiefstpunkt 109 der zweiten Stentfeder 102 einem Scheitelpunkt 108 der distal gelegenen Stentfeder 103 direkt zugeordnet, so dass diese beiden Punkte der beiden Stentfedern 102, 103 sich gegenüberliegen, wobei auch hier wiederum ein Abstand zwischen den beiden Stentfedern 102, 103 vorliegt. Aufgrund dieser Anordnung ergibt sich ein insgesamt symmetrisches Muster bzw. eine symmetrische Anordnung der in Längsrichtung des Gefäßimplantats 100 angeordneten Stentfedern 101, 102, 103, 104, 105, 106.

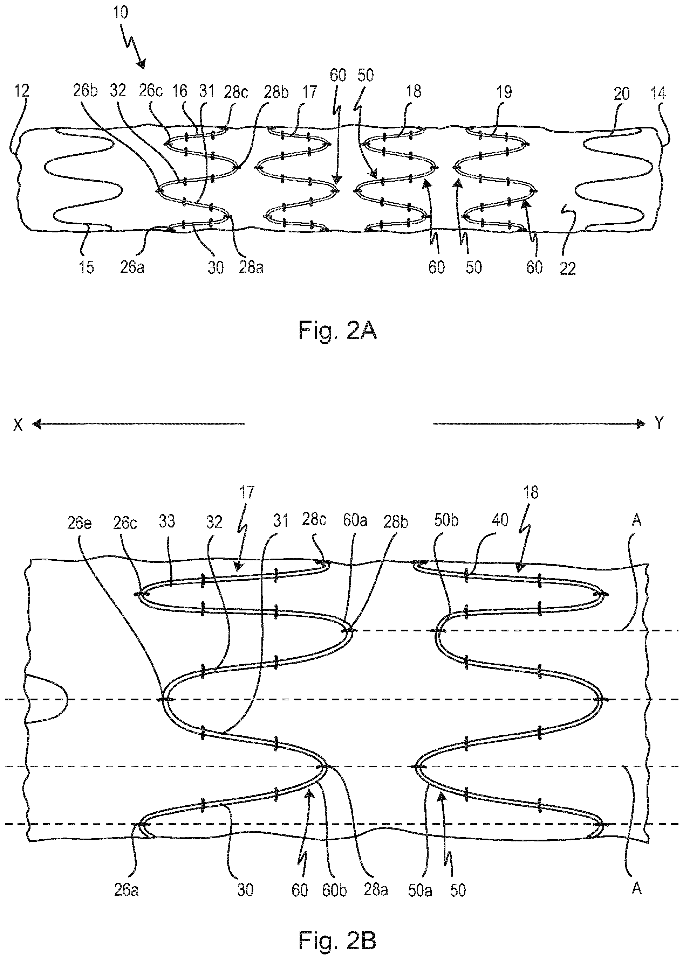

Nachfolgend werden Ausführungsformen des erfindungsgemäßen Gefäßimplantats beschrieben. Eine Ausführungsform ist in den Abbildungen 2a und 2b schematisch und nicht maßstabsgetreu dargestellt.

Die in Fig. 2a dargestellte Ausführungsform des erfindungsgemäßen Gefäßimplantats 10 besitzt ein proximales Ende 12 und ein distales Ende 14, und weist in Längsrichtung hintereinander angeordnete Stentfedern 15, 16, 17, 18, 19, 20 auf, die jeweils nicht direkt/unmittelbar miteinander verbunden sind, sondern lediglich über das Implantatmaterial 22, so dass ein hohlzylindrischer Körper 24 gebildet wird.

Die Stentfedern 15 bis 20 sind jeweils einstückig und umlaufend wellen- bzw. schleifenbildend ausgebildet, und erstrecken sich somit mäanderförmig über den Umfang des Gefäßimplantats 10. Auch in Fig. 2 ist die proximale Richtung mit x und die distale Richtung mit y, sowie über in die jeweilige Richtung weisende Pfeile gekennzeichnet. Erfindungsgemäß weisen die Stentfedern 15, 16, 17, 18, 19, 20 des Gefäßimplantats 10 in proximale Richtung x weisende Spitzbögen 50 sowie in distale Richtung y weisende Spitzbögen 60 auf, wobei aus Gründen der Übersichtlichkeit nicht alle Spitzbögen mit Bezugszeichen versehen sind, sondern leidglich und beispielhaft jeweils zwei Spitzbögen 50 und 60 der Stentfedern 18 und 19. Die Spitzbögen 50, 60 der Stentfedern bilden anders gesprochen umlaufende Wellen mit Wellenbergen, die den Spitzbögen 50 entsprechen, und Wellentälern, die den Spitzbögen 60 entsprechen.

Die einzelnen Stentfedern 15 bis 20 weisen jeweils Scheitelpunkte 26a, 26b, 26c und Tiefstpunkte 28a, 28b, 28c auf, wobei mit "Scheitelpunkten" vorliegend jeweils der höchste Punkt einer Welle/des Wellenbergs, betrachtet in proximaler Richtung, bezeichnet wird, und mit "Tiefstpunkt " der jeweils tiefste Punkt einer Welle/des Wellentals. Auch hier wurden aus Gründen der Übersichtlichkeit nicht alle Scheitelpunkte und Tiefstpunkte aller Stentfedern mit Bezugszeichen versehen. Die Scheitelpunkte 26a, 26b, 26c stellen also jeweils den höchsten Punkt der in proximale Richtung x weisenden Spitzbögen 50 dar, und die Tiefstpunkte 28a, 28b, 28c jeweils den höchsten Punkt der in distale Richtung y weisenden Spitzbögen 60.

Wie Fig. 2 zu entnehmen ist, sind die Stentfedern 16, 17, 18, 19 an das Implantatmaterial 22 über Nähte 40 angenäht.

Grundsätzlich werden - wie bereits weiter oben beschrieben - bei selbstexpandierenden Gefäßimplantaten bzw. Stentgrafts oder endoluminalen Implantaten, allgemein sowie vorliegend zur Bezeichnung der jeweiligen Enden der Gefäßimplantate die Begriffe "distal" und "proximal" verwendet, wobei der Begriff "distal" den Teil bzw. das Ende bezeichnet, der/das in Bezug auf den Blutstrom weiter stromabwärts liegt. Der Begriff "proximal" hingegen bezeichnet, wieder in Bezug auf den Blutstrom, einen Teil bzw. das Ende, der/das in Bezug auf den Blutstrom weiter stromaufwärts liegt. Anders ausgedrückt bedeutet der Begriff "distal" in Richtung des Blutstrom, und der Begriff "proximal" der Richtung des Blutstroms entgegengesetzt. Bei Kathetern hingegen, bzw. Einführsystemen für selbstexpandierende Gefäßimplantate bezeichnet der Begriff "distal" das Ende des Katheters bzw. Einführsystems, das in den Patienten eingeführt wird, bzw. das vom Anwender des Katheters/Einführsystems ausgesehen am entferntesten liegt; und der Begriff "proximal" bezeichnet das Ende des Katheters/Einführsystems, das dem Anwender näher zugewandt ist.

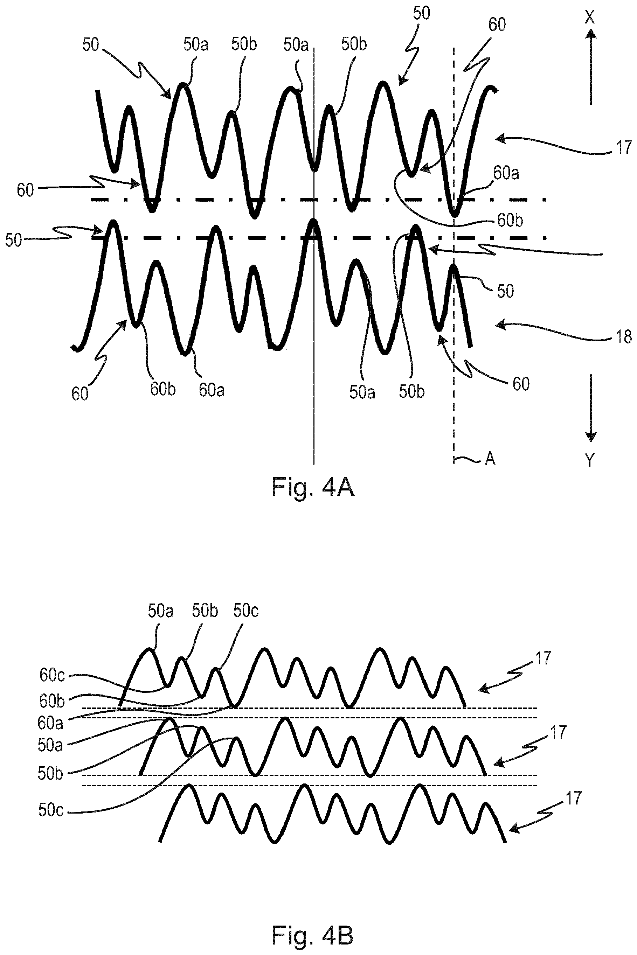

Fig. 2a, und in vergrößerter, ausschnittsweiser Darstellung Fig. 2b, ist zu entnehmen, dass zumindest eine der Stentfedern 15 bis 20 Schenkel 30, 31, 32, 33 aufweist, die - respektive - einen ersten Tiefstpunkt 28a mit einem ersten Scheitelpunkt 26a, diesen wieder mit einem zweiten Tiefstpunkt 28b, diesen Tiefstpunkt 28b mit einem zweiten Scheitelpunkt 26b, und diesen Scheitelpunkt 26b mit einem dritten Tiefstpunkt 28c verbinden, und diesen wiederum mit dem Scheitelpunkt 26c. Dabei besitzen die Schenkel 30, 31, 32, voneinander unterschiedliche Längen, wodurch wiederum Spitzbögen 50, 60 mit unterschiedlichen Höhen gebildet werden (siehe auch Fig. 4). Dadurch wird insgesamt ein asymmetrischer Verlauf der Spitzbögen bzw. "Wellen" der Stentfeder 15, 16, 17, 18, 19, 20 bewirkt, der zu der verminderten Knickbildung beiträgt.

Den Figuren 2a und 2b ist ferner zu entnehmen, das die Stentfedern 17 und 18 derart zueinander angeordnet sind, dass ein in proximale Richtung x weisender höherer Spitzbogen 50a einer ersten Stentfeder 18 einem in distale Richtung y weisenden kürzeren Spitzbogen 60b einer in proximale Richtung x nachgeordneten zweiten Stentfeder 17 in einem Abstand und in einer gedachten Linie (A) gegenüber liegt, die parallel zur Längsrichtung des Gefäßimplantats 10 ist. Ebenso liegt ein in proximale Richtung x weisender kürzerer Spitzbogen 50b einer ersten Stentfeder 18 einem in distale Richtung y weisenden höherem Spitzbogen 60a einer in proximale Richtung x nachgeordneten zweiten Stentfeder 17 in einem Abstand und in einer gedachten Linie (A) gegenüber liegt, die parallel zur Längsrichtung des Gefäßimplantats 10 ist.

Durch diese Anordnung erhält das Gefäßimplantat - in Zusammenwirkung mit dem asymmetrischen mäandrischen Verlauf der einzelnen Stentfedern 15, 16, 17, 18, 19, 20 - insgesamt auch eine asymmetrische Anordnung bezüglich der einzelnen Stentfedern 15, 16, 17, 18, 19, 20 untereinander, und diese weitere asymmetrische Ausbildung verstärkt den Effekt der Knickbildungsvermeidung weiter.

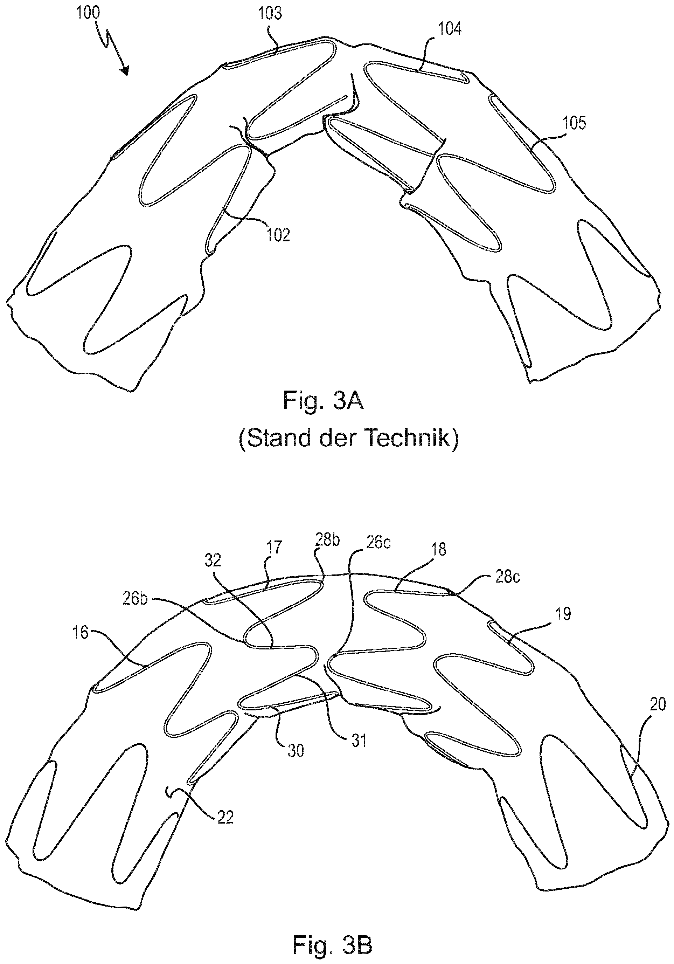

Der Effekt der Vermeidung der Knickbildung ist in Fig. 3 anschaulich dargestellt: Hier ist in Fig. 3a ein im Stand der Technik bekanntes Gefäßimplantat 100 gezeigt, das in einen gebogenen Zustand versetzt wurde. Das im Stand der Technik bekannte Gefäßimplantat 100 weist an der Biegung eine starke Knickstelle auf, die den Blutfluss durch das Gefäß maßgeblich beeinträchtigen kann.

Hingegen zeigt die erfindungsgemäße Ausführungsform des Gefäßimplantats 10, wie sie in Fig. 3b dargestellt ist, im gebogenen Zustand keine Knickbildung, hier bilden sich lediglich ggf. höchstens kleine Fältchen im Implantatmaterial 22, jedoch kein Knick zwischen zwei hintereinander angeordneten Stentfedern 15, 16, 17, 18, 19, 20, wie es im Fall des im Stand der Technik bekannten Gefäßimplantats 100 ist.

Fig. 4 zeigt darüber hinaus zwei beispielhafte schematische Ausbildungen und Anordnungen zweier (bzw. dreier) nachgelagerter Stentfedern 17 und 18 (Fig. 4A) bzw. 17, 18 und 19 (Fig. 4B). Hierbei werden die gleichen Bezugszeichen zur Benennung gleicher Merkmale wie in den Figuren 1 bis 3 verwendet.

Fig. 4A ist der asymmetrische mäandrische Verlauf der beiden Stentfedern 17 und 18 der beispielhaften Ausführungsform des erfindungsgemäßen Gefäßimplantats zu entnehmen: Die Stentfedern 17 und 18 weisen abwechselnd bzw. alternierend in proximale Richtung x weisende Spitzbögen 50 und in distale Richtung y weisende Spitzbögen 60 auf. Die in proximale Richtung x weisenden Spitzbögen 50 umfassen abwechselnd hohe bzw. höherer Spitzbögen 50a und kürzere Spitzbögen 50b, die kürzer als die jeweils davor angeordneten, in proximale Richtung x weisenden höheren Spitzbögen 50a sind. Ferner umfassen die Stentfedern 17 und 18 jeweils in distale Richtung y weisende Spitzbögen 60, mit höheren Spitzbögen 60a und kürzeren Spitzbögen 60b. Dabei sind die beiden Stentfedern 17, 18 bezüglich Ihrer Spitzbögen derart angeordnet, dass ein in distale Richtung x weisender höherer Spitzbogen 60a der Stentfeder 17 einem kürzeren Spitzbogen 50b der in distaler Richtung y nachgeordneten Stentfeder 18 in einem Abstand und einer gedachten Linie A gegenüber liegt, die parallel zur Längsachse des Gefäßimplantats ist, bzw. senkrecht zum Umlauf der Stentfeder 17, 18.

Fig. 4B ist eine weitere Ausführungsform zu entnehmen: Hier wechseln sich drei unterschiedlich hohe, in proximale Richtung x weisende Spitzbögen derart ab, dass auf einen höheren weisenden Spitzbogen 50a ein kürzerer Spitzbogen 50b, und auf diesen ein Spitzbogen 50c anschließt, der noch kürzer als der Spitzbogen 50b ist; an diesen schließt sich wiederum ein höherer Spitzbogen 50a an, auf welchen wieder ein kürzerer Spitzbogen 50b folgt, usw. Aufgrund dieser Ausbildung sind die in distale Richtung y weisenden Spitzbögen 60 in umlaufender Richtung der Stentfeder 17 derart, dass auf einen kürzeren Spitzbogen 60c, ein Spitzbogen 60b folgt, der höher als der Spitzbogen 60c ist, und auf diesen Spitzbogen 60b ein höherer Spitzbogen 60a, der höher als der Spitzbogen 60b ist. Die Spitzbögen 50b und 60b weisen daher jeweils Höhen auf, die zwischen den Höhen des höheren und des kürzeren Spitzbogens 50a und 50c bzw. 60a und 60c liegen

Bei dem erfindungsgemäßen Gefäßimplantat sind die beiden Stentfedern 17 und 18 dann derart nacheinander angeordnet, dass der höhere, in proximale Richtung weisende Spitzbogen 50a der Stentfeder 18 dem kürzeren, in distale Richtung weisenden Spitzbogen 60c der Stentfeder 17 gegenüber liegt, der Spitzbogen 50b der Stentfeder 18 dem Spitzbogen 60b und der kürzere, in proximale Richtung weisende Spitzbogen 50c der Stentfeder 18 dem in distale Richtung weisenden höheren Spitzbogen 60a der Stentfeder 17. Durch die in Fig. 4B gezeigten und hintereinander schematisch angeordneten Stentfedern 17, 18 und 19 lässt sich die insgesamt asymmetrische Anordnung gut erkennen.

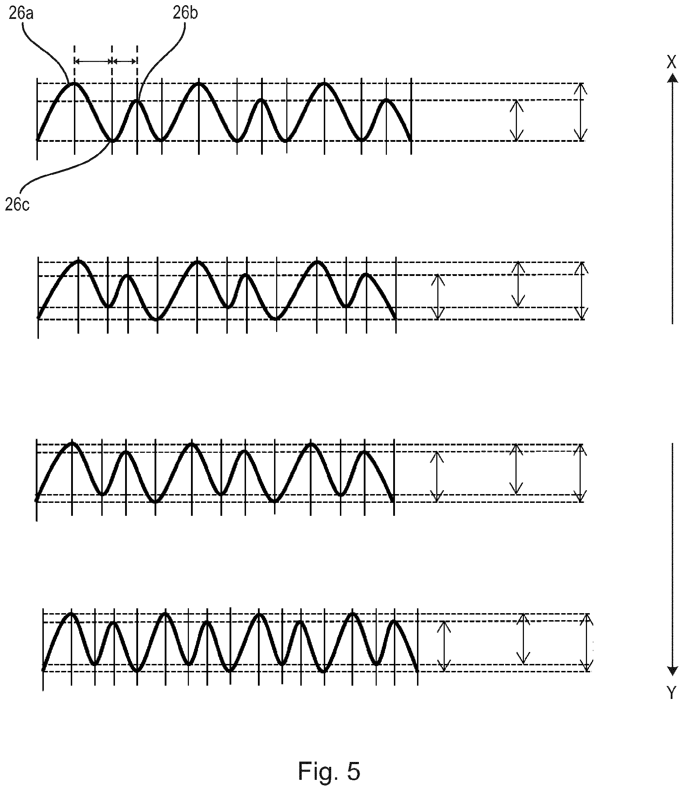

Fig. 5 zeigt schließlich verschiedene Ausbildungen von im Sinne der vorliegenden Anmeldung geeigneten Stentfedern, mit asymmetrisch ausgebildeten Spitzbögen mit unterschiedlichen Höhen. Bei der erfindungsgemäßen asymmetrischen Anordnung solcher Stentfedern im Verhältnis zueinander und in nachgeordneter Reihenfolge lässt sich in Kombination mit den einzelnen asymmetrischen Stentfedern ein Gefäßimplantat bereitstellen, mit dem eine Knickbildung in einem Gefäß, wie vorliegend beschrieben, vorteilhaft vermieden werden kann.

Fig. 5 zeigt vier beispielhafte Verläufe der Stentfedern, wobei der oberste Verlauf nicht unter den Schutzbereich fällt; die unteren drei Ausführungen hingegen schon. Die in Fig. 5 in den drei unteren Ausführungen dargestellten Verläufe beispielhafter Stentfedern zeigen unterschiedliche Variationen und Möglichkeiten zur Ausbildung asymmetrischer Stentfedern, wobei in den oberen drei jeweils zwei unterschiedliche Höhen der in die proximale Richtung x und in die distale Richtung y weisenden Spitzbögen, wohingegen die Stentfeder ganz unten in Fig. 5 jeweils drei unterschiedliche Höhen der Spitzbögen aufweist.

Ferner ist aus den beiden oberen in Fig. 5 gezeigten Stentfedern zu erkennen, dass die beiden unterschiedlichen Höhen der jeweils in distale und proximale Richtung weisenden Spitzbögen auch unterschiedliche Werte haben können: So ist in dem in Fig. 5 gezeigten obersten Beispiel der Unterschied zwischen den Höhen der Spitzbögen größer als in dem zweitobersten Beispiel.

Außerdem kann durch den Abstand der in proximale x bzw. distale y Richtung weisenden Spitzbögen die Anzahl der Spitzbögen einer Stentfeder geregelt werden: je enger der Abstand, desto mehr Spitzbögen kann eine Stentfeder aufweisen, was insbesondere aus dem dritten Beispiel hervorgeht.

Schließlich ist in dem untersten Beispiel ein Verlauf mit jeweils drei unterschiedlichen Höhen der in proximale bzw. distale Richtungen weisenden Spitzbögen gezeigt.

Dem obersten Beispiel in Fig. 5 ist ferner zu entnehmen, dass sich hier der Abstand z1 eines ersten Scheitelpunkts 26a zu einem zu einem auf den ersten Scheitelpunkt 26a in umlaufender Richtung folgenden erster Tiefstpunkt 28a zumindest einer Stentfeder unterscheidet von dem Abstand z2 des ersten Tiefstpunkts 28a zu einem auf den ersten Tiefstpunkt 28a in umlaufender Richtung folgenden zweiten Scheitelpunkt 26b derselben Stentfeder. Dadurch keine eine weitere Asymmetrie erreicht werden.

## Patentansprüche

1. Selbstexpandierendes Gefäßimplantat (10) zur Implantation in ein Blutgefäß mit einem hohlzylindrischen Körper (24) mit einem proximalen (12) und einem distalen Ende (14) und einer Längsrichtung, sowie mit in seiner Längsrichtung in einem Abstand hintereinander angeordneten, jeweils mäanderförmig umlaufenden Stentfedern (15, 16, 17, 18, 19, 20) und einem an den Stentfedern (15, 16, 17, 18, 19, 20) befestigten und diese verbindenden Implantatmaterial (22), wobei die Stentfedern (15, 16, 17, 18, 19, 20) lediglich über das Implantatmaterial (22) und nicht untereinander verbunden sind, und wobei die umlaufenden Stentfedern (15, 16, 17, 18, 19, 20) abwechselnd in proximale (x) und distale (y) Richtung weisende Spitzbögen aufweisen, welche über Schenkel miteinander verbunden sind, wobei ein in proximale Richtung x weisender höherer Spitzbogen (50a) einer ersten Stentfeder (18) einem in distale Richtung y weisenden kürzeren Spitzbogen (60b) einer proximal nachgeordneten zweiten Stentfeder (17) in einem Abstand und in einer gedachten Linie (A) gegenüber liegt, die parallel zur Längsrichtung des Gefäßimplantats (10) ist, und/oder wobei ein in proximale Richtung x weisender kürzerer Spitzbogen (50b) einer ersten Stentfeder (18) einem in distale Richtung y weisenden längeren Spitzbogen (60a) einer in proximale Richtung x nachgeordneten zweiten Stentfeder (17) in einem Abstand und in einer gedachten Linie (A) gegenüber liegt, die parallel zur Längsrichtung des Gefäßimplantats (10) ist, **dadurch gekennzeichnet, dass** zumindest zwei in Längsrichtung hintereinander angeordnete Stentfedern (15, 16, 17, 18, 19, 20) jeweils Schenkel (30, 31, 32, 33) mit unterschiedlichen Längen aufweisen zur Ausbildung von umlaufend aufeinander folgenden unterschiedlich hohen Spitzbögen (50, 60), mit höheren (50a) und kürzeren (50b) Spitzbögen, wobei zumindest drei in umlaufender Richtung unmittelbar aufeinanderfolgende Schenkel (30, 31, 32, 33) einer Stentfeder (15, 16, 17, 18, 19, 20) jeweils unterschiedliche Längen aufweisen.

2. Selbstexpandierendes Gefäßimplantat nach Anspruch 1, **dadurch gekennzeichnet, dass** in umlaufender Richtung der Stentfeder (15, 16, 17, 18, 19, 20) jeweils ein höherer (50a) mit einem kürzeren (50b) in proximale Richtung x weisenden Spitzbogen alterniert, und jeweils ein höherer (60a) mit einem kürzeren (60b) in distale Richtung y weisender Spitzbogen.

3. Selbstexpandierendes Gefäßimplantat (10) nach einem der Ansprüche 1 oder 2, **dadurch gekennzeichnet, dass** bei zumindest einer Stentfeder (15, 16, 17, 18, 19, 20) in umlaufender Richtung die in proximale Richtung x weisenden Spitzbögen (50) derart ausgebildet sind, dass auf einen hohen Spitzbogen (50a) ein kürzerer Spitzbogen (50b) folgt, und auf den kürzeren Spitzbogen (50b) ein Spitzbogen (50c), der kürzer ist als der kürzere Spitzbogen (50b), und auf diesen wieder ein hoher Spitzbogen (50a).

4. Selbstexpandierendes Gefäßimplantat (10) nach Anspruch 1, **dadurch gekennzeichnet, dass** bei zumindest einer Stentfeder (15, 16, 17, 18, 19, 20) in umlaufender Richtung die in proximale Richtung x weisenden Spitzbögen (50) alle die gleiche Höhe aufweisen, welche sich mit in distale Richtung y weisenden Spitzbögen (60) unterschiedlicher Höhe (60a, 60b) abwechseln.

5. Selbstexpandierendes Gefäßimplantat (10) nach Anspruch 1, **dadurch gekennzeichnet, dass** bei zumindest einer Stentfeder (15, 16, 17, 18, 19, 20) in umlaufender Richtung die in distale Richtung y weisenden Spitzbögen (60) alle die gleiche Höhe aufweisen, welche sich mit in proximale Richtung x weisenden Spitzbögen (50) unterschiedlicher Höhe (50a, 50b) abwechseln

6. Selbstexpandierendes Gefäßimplantat (10) nach einem der Ansprüche 1 bis 5, **dadurch gekennzeichnet, dass** sich der Abstand z1 eines ersten Scheitelpunkts (26a) zu einem auf den ersten Scheitelpunkt (26a) in umlaufender Richtung folgender erster Tiefstpunkt (28a) zumindest einer Stentfeder (15, 16, 17, 18, 19; 20) unterscheidet von dem Abstand z2 des ersten Tiefstpunkts (28a) zu einem auf den ersten Tiefstpunkt (28a) in umlaufender Richtung folgenden zweiten Scheitelpunkt (26b) derselben Stentfeder (15, 16, 17, 18, 19; 20).

7. Selbstexpandierendes Gefäßimplantat (10) nach einem der vorstehenden Ansprüche, **dadurch gekennzeichnet, dass** es zwischen 6 und 12 in proximale Richtung x weisende Spitzbögen (50) und zwischen 6 und 12 in distale Richtung y weisende Spitzbögen (60) aufweist.

8. Selbstexpandierendes Gefäßimplantat (10) nach einem der vorstehenden Ansprüche, **dadurch gekennzeichnet, dass** es zumindest drei in Längsrichtung hintereinander angeordnete Stentfedern (15, 16, 17, 18, 19, 20) aufweist.

9. Selbstexpandierendes Gefäßimplantat (10) nach einem der vorstehenden Ansprüche, **dadurch gekennzeichnet, dass** es zwischen drei und zehn hintereinander angeordneten Stentfedern (15, 16, 17, 18, 19, 20) aufweist.

10. Selbstexpandierendes Gefäßimplantat (10) nach einem der vorstehenden Ansprüche, **dadurch gekennzeichnet, dass** die Stentfedern (15, 16, 17, 18, 19, 20) an das Implantatmaterial in einem Abstand voneinander angenäht sind.

11. Selbstexpandierendes Gefäßimplantat (10) nach einem der vorstehenden Ansprüche, **dadurch gekennzeichnet, dass** es als Gefäßimplantat (10) mit einem zumindest einem, von dem hohlzylindrischen Körper (24) abzweigenden Seitenkörper ausgebildet ist.

## Claims

1. A self-expanding vascular implant (10) for implanting into a blood vessel, comprising a hollow-cylindrical body (24) with a proximal end (12) and a distal end (14) and a longitudinal direction, and also comprising stent springs (15, 16, 17, 18, 19, 20) successively arranged at a distance in its longitudinal direction and, respectively, circumferentially meandering, and comprising an implant material (22) fixed to the stent springs (15, 16, 17, 18, 19, 20) and connecting them, the stent springs (15, 16, 17, 18, 19, 20) only being connected by way of the implant material (22) and not between one another, and the circumferential stent springs (15, 16, 17, 18, 19, 20) having pointed arches facing alternately toward the proximal direction (x) and the distal direction (y), which are connected to one another by way of legs, wherein a higher pointed arch (50a), facing toward the proximal direction x, of a first stent spring (18) lies opposite a shorter pointed arch (60b), facing toward the distal direction y, of a second stent spring (17) arranged proximally thereafter, at a distance and at an imaginary line (A) that is parallel to the longitudinal direction of the vascular implant (10), and/or in that a shorter pointed arch (50b), facing toward the proximal direction x, of a first stent spring (18) lies opposite a longer pointed arch (60a), facing toward the distal direction y, of a second stent spring (17) arranged thereafter in the proximal direction x, at a distance and at an imaginary line (A) that is parallel to the longitudinal direction of the vascular implant (10) **characterized in that** at least two stent springs (15, 16, 17, 18, 19, 20) successively arranged in the longitudinal direction, respectively, have legs (30, 31, 32, 33) of different lengths for forming of circumferentially arranged pointed arches (50, 60) of varying height, with higher (50a) and shorter (50b) pointed arches, wherein at least three legs (30, 31, 32, 33) that are successively arranged in circumferential direction of one stent spring (15, 16, 17, 18, 19, 20) have different lengths.

2. The self-expanding vascular implant as claimed in claim 1, **characterized in that**, in the circumferential direction of the stent spring (15, 16, 17, 18, 19, 20), a higher pointed arch (50a) facing toward the proximal direction x respectively alternates with a shorter pointed arch (50b) facing toward the proximal direction x, and a higher pointed arch (60a) facing toward the distal direction y respectively alternates with a shorter pointed arch (60b) facing toward the distal direction y.

3. The self-expanding vascular implant (10) as claimed in claim 1 or 2, **characterized in that** in at least one stent spring (15, 16, 17, 18, 19, 20), in the circumferential direction, the pointed arches (50) facing toward the proximal direction x are formed such, that a high pointed arch (50a) is followed by a shorter pointed arch (50b), and the shorter pointed arch (50b) is followed by a pointed arch (50c) that is shorter than the shorter pointed arch (50b), and this is again followed by a high pointed arch (50a).

4. The self-expanding vascular implant (10) as claimed in claim 1, **characterized in that** in at least one stent spring (15, 16, 17, 18, 19, 20), in the circumferential direction, the pointed arches (50) facing toward the proximal direction x are all of the same height and alternate with pointed arches (60) of varying height (60a, 60b) facing toward the distal direction y.

5. The self-expanding vascular implant (10) as claimed in claim 1, **characterized in that** in at least one stent spring (15, 16, 17, 18, 19, 20), in the circumferential direction, the pointed arches (60) facing in the distal direction y are all of the same height and alternate with pointed arches (50) of varying height (50a, 50b) facing toward the proximal direction x.

6. The self-expanding vascular implant (10) as claimed in one of claims 1 to 5, **characterized in that** the distance z1 between a first vertex (26a) and a first lowest point (28a) following the first vertex (26a) in the circumferential direction of at least one stent spring (15, 16, 17, 18, 19; 20) differs from the distance z2 between the first lowest point (28a) and a second vertex (26b) following the first lowest point (28a) in the circumferential direction of the same stent spring (15, 16, 17, 18, 19; 20).

7. The self-expanding vascular implant (10) as claimed in one of the preceding claims, **characterized in that** it has between 6 and 12 pointed arches (50) facing toward the proximal direction x and between 6 and 12 pointed arches (60) facing toward the distal direction y.

8. The self-expanding vascular implant (10) as claimed in one of the preceding claims, **characterized in that** it has at least three stent springs (15, 16, 17, 18, 19, 20) successively arranged one behind the other in the longitudinal direction.

9. The self-expanding vascular implant (10) as claimed in one of the preceding claims, **characterized in that** it has between three and ten stent springs (15, 16, 17, 18, 19, 20) successively arranged one behind the other.

10. The self-expanding vascular implant (10) as claimed in one of the preceding claims, **characterized in that** the stent springs (15, 16, 17, 18, 19, 20) are sewn onto the implant material at a distance from one another.

11. The self-expanding vascular implant (10) as claimed in one of the preceding claims, **characterized in that** it is formed as a vascular implant (10) comprising a at least one side body branching off from the hollow-cylindrical body (24).

## Revendications

1. Implant vasculaire auto-expansé (10) destiné à être implanté dans un vaisseau sanguin, comportant un corps cylindrique creux (24) présentant une extrémité proximale (12) et une extrémité distale (14) et une direction longitudinale, et comportant des ressorts de stent (15, 16, 17, 18, 19, 20) disposés les uns derrière les autres dans la direction longitudinale et s'étendent respectivement de manière périphérique en forme de méandres et un matériau d'implant (22) fixé aux ressorts de stent (15, 16, 17, 18, 19, 20) et reliant ceux-ci les uns aux autres, dans lequel les ressorts de stent (15, 16, 17, 18, 19, 20) sont reliés seulement par le biais du matériau d'implant (22) et ne sont pas reliés les uns aux autres, et dans lequel les ressorts de stent périphériques (15, 16, 17, 18, 19, 20) comprennent des portions courbées en pointe orientées en alternance dans la direction proximale (x) et dans la direction distale (y), lesquelles sont reliées les unes aux autres par le biais de branches, dans lequel une portion courbée en pointe plus haute (50a), orientée dans la direction proximale x, d'un premier ressort de stent (18) est en regard d'une portion courbée en pointe plus courte (60b), orientée dans la direction distale y, d'un deuxième ressort de stent (17) situé en aval dans la direction proximale, à une distance et suivant une ligne imaginaire (A) qui est parallèle à la direction longitudinale de l'implant vasculaire (10), et/ou dans lequel une portion courbée en pointe plus courte (50b), orientée dans la direction proximale x, d'un premier ressort de stent (18) est en regard d'une portion courbée en pointe plus longue (60a), orientée dans la direction distale y, d'un deuxième ressort de stent (17) situé en aval dans la direction proximale x, à une distance et suivant une ligne imaginaire (A) qui est parallèle à la direction longitudinale de l'implant vasculaire (10), **caractérisé en ce qu'**au moins deux ressorts de stent (15, 16, 17, 18, 19, 20) disposés les uns derrière les autres dans la direction longitudinale comprennent respectivement des branches (30, 31, 32, 33) de longueurs différentes pour la formation de portions courbées en pointe (50, 60) de hauteurs différentes, se suivant sur la périphérie, avec des portions courbées en pointe plus hautes (50a) et plus courtes (50b), au moins trois branches (30, 31, 32, 33), se suivant directement dans la direction périphérique, d'un ressort de stent (15, 16, 17, 18, 19, 20) présentant respectivement des longueurs différentes.

2. Implant vasculaire auto-expansé selon la revendication 1, **caractérisé en ce que**, dans la direction périphérique des ressorts de stent (15, 16, 17, 18, 19, 20), respectivement une portion courbée en pointe plus haute (50a) alterne avec une portion courbée en pointe plus courte (50b) orientée dans la direction proximale x, et respectivement une portion courbée en pointe plus haute (60a) alterne avec une portion courbée en pointe plus courte (60b) orientée dans la direction distale y.

3. Implant vasculaire auto-expansé (10) selon l'une des revendications 1 ou 2, **caractérisé en ce que** dans le cas d'au moins un ressort de stent (15, 16, 17, 18, 19, 20), les portions courbées en pointe (50) orientées dans la direction proximale x sont formées dans la direction périphérique de telle sorte qu'une portion courbée en pointe plus courte (50b) suive une portion courbée en pointe haute (50a), et qu'une portion courbée en pointe (50c) qui est plus courte que la portion courbée en pointe plus courte (50b) suive la portion courbée en pointe plus courte (50b), et qu'une portion courbée en pointe haute (50a) suis à nouveau celle-ci.

4. Implant vasculaire auto-expansé (10) selon la revendication 1, **caractérisé en ce que** dans le cas d'au moins un ressort de stent (15, 16, 17, 18, 19, 20), les portions courbées en pointe (50) orientées dans la direction proximale x présentent toutes la même hauteur dans la direction périphérique, lesquelles alternent avec des portions courbées en pointe (60), orientées dans la direction distale y, d'une hauteur différente (60a, 60b).

5. Implant vasculaire auto-expansé (10) selon la revendication 1, **caractérisé en ce que** dans le cas d'au moins un ressort de stent (15, 16, 17, 18, 19, 20), les portions courbées en pointe (60) orientées dans la direction distale y présentent toutes la même hauteur dans la direction périphérique, lesquelles alternent avec des portions courbées en pointe (50), orientées dans la direction proximale x, d'une hauteur différente (50a, 50b).

6. Implant vasculaire auto-expansé (10) selon l'une des revendications 1 à 5, **caractérisée en ce que** la distance z1 d'un premier sommet (26a) à un point le plus bas (28a), suivant le premier sommet (26a) dans la direction périphérique, d'au moins un ressort de stent (15, 16, 17, 18, 19; 20) diffère de la distance z2 du premier point le plus bas (28a) à un deuxième sommet (26b), suivant le premier point le plus bas (28a) dans la direction périphérique, du même ressort de stent (15, 16, 17, 18, 19; 20).

7. Implant vasculaire auto-expansé (10) selon l'une des revendications précédentes, **caractérisé en ce qu'**il comprend entre 6 et 12 portions courbées en pointe (50) orientées dans la direction proximale x et entre 6 et 12 portions courbées en pointe (60) orientées dans la direction distale y.

8. Implant vasculaire auto-expansé (10) selon l'une des revendications précédentes, **caractérisé en ce qu'**il comprend au moins trois ressorts de stent (15, 16, 17, 18, 19, 20) disposés les uns derrière les autres dans la direction longitudinale.

9. Implant vasculaire auto-expansé (10) selon l'une des revendications précédentes, **caractérisé en ce qu'**il comprend entre trois et dix ressorts de stent (15, 16, 17, 18, 19, 20) disposés les uns derrière les autres.

10. Implant vasculaire auto-expansé (10) selon l'une des revendications précédentes, **caractérisé en ce que** les ressorts de stent (15, 16, 17, 18, 19, 20) sont cousus sur le matériau d'implant à distance les uns des autres.

11. Implant vasculaire auto-expansé (10) selon l'une des revendications précédentes, **caractérisé en ce qu'**il est réalisé comme un implant vasculaire (10) comportant au moins un corps latéral bifurquant à partir du corps cylindrique creux (24).
